# EUROPEAN PATENT APPLICATION

(11) **EP 4 488 355 A1**
(43) Date of publication of application: **08.01.2025**
(21) Application number: 23778968.0
(22) Date of filing: 15.02.2023
(51) Int. Cl.: C12M 1/00, C12M 3/00, C12P 21/02, C12P 21/08

(54) **METHOD FOR PRODUCING ACTIVE PHARMACEUTICAL INGREDIENT OF BIOPHARMACEUTICAL, SYSTEM FOR PRODUCING ACTIVE PHARMACEUTICAL INGREDIENT OF BIOPHARMACEUTICAL, AND ACTIVE PHARMACEUTICAL INGREDIENT OF BIOPHARMACEUTICAL**

(30) Priority: 30.03.2022 JP 2022057524
(71) Applicant: FUJIFILM Corporation, Tokyo 106-8620 (JP)
(72) Inventor: NAKAI, Shinichi, Ashigarakami-gun, Kanagawa 258-8577 (JP); SAKUYAMA, Hiroshi, Ashigarakami-gun, Kanagawa 258-8577 (JP); TAKAHASHI, Naoto, Ashigarakami-gun, Kanagawa 258-8577 (JP)
(74) Representative: Hoffmann Eitle
(86) International application number: PCT/JP2023/005301
(87) International publication number: WO 2023/188937

(57) **Abstract**

Provided is a method for producing an active pharmaceutical ingredient of a biopharmaceutical, the method including: connecting, through a coupling line, a culture section where a culture step, in which cells are cultured in a culture liquid stored in a culture tank and the cells are removed from the culture liquid to obtain a culture supernatant liquid containing a product of the cells, is performed and a purification section where a purification step, in which the product is purified from the culture supernatant liquid to obtain an active pharmaceutical ingredient of a biopharmaceutical, is performed, to allow the culture supernatant liquid to flow into the purification section from the culture section through the coupling line; connecting a flexible single-use tank having an accommodation capacity of 0.2 times to 10 times an amount of the culture liquid in the culture tank to a branch line provided between the culture section and the purification section; and temporarily storing the culture supernatant liquid in the tank.

## Description

### BACKGROUND OF THE INVENTION

### 1. Field of the Invention

The technology of the present disclosure relates to a method for producing an active pharmaceutical ingredient of a biopharmaceutical, a system for producing an active pharmaceutical ingredient of a biopharmaceutical, and an active pharmaceutical ingredient of a biopharmaceutical.

### 2. Description of the Related Art

A cell product such as an antibody is obtained by culturing a cell such as an antibody-producing cell in which an antibody gene is incorporated into Chinese Hamster Ovary (CHO) cells, and an active pharmaceutical ingredient of a biopharmaceutical is obtained by purifying the product. In production of such an active pharmaceutical ingredient of a biopharmaceutical, in the related art, batch production in which a cell culture step and a cell product purification step are separately performed has been performed, but in recent years, continuous production has become mainstream instead of the batch production. The continuous production is a method in which a culture section where a culture step is performed, which includes a culture tank and the like, and a purification section where a purification step is performed, which includes a chromatography device and the like, are connected to each other physically, and the culture step and the purification step are continuously and consistently performed. According to the continuous production, quality and productivity of the active pharmaceutical ingredient can be significantly improved as compared with the batch production.

JP2017-515501A discloses a system for producing an active pharmaceutical ingredient of a biopharmaceutical by the continuous production. In the production system disclosed in JP2017-515501A, in addition to a coupling line which connects the culture section and the purification section, a branch line is provided between the culture section and the purification section, and a tank (container m in Fig. 5 of JP2017-5 15501A) is connected to the branch line.

### SUMMARY OF THE INVENTION

From Fig. 5, it can be seen that the tank disclosed in JP2017-515501A is made of glass repeatedly used. Therefore, there is a concern about contamination of a culture supernatant liquid. In addition, in a case where one tank is exchanged with a new tank when the one tank is filled with the culture supernatant liquid, since the glass is heavy, there is a disadvantage that it is difficult to handle the glass because of the time and effort required for sterilization. Such problems of contamination and difficulty in handling are more likely to be manifested as an accommodation capacity of the tank increases. The culture supernatant liquid is obtained by removing cells from a culture liquid, and contains a cell product. The culture supernatant liquid flows from the culture section to the purification section through the coupling line.

One embodiment according to the technology of the present disclosure provides a method for producing an active pharmaceutical ingredient of a biopharmaceutical, which is capable of reducing a concern of contamination and is easy to handle; a system for producing an active pharmaceutical ingredient of a biopharmaceutical; and an active pharmaceutical ingredient of a biopharmaceutical.

A method for producing an active pharmaceutical ingredient of a biopharmaceutical according to the present disclosure includes connecting, through a coupling line, a culture section where a culture step, in which cells are cultured in a culture liquid stored in a culture tank and the cells are removed from the culture liquid to obtain a culture supernatant liquid containing a product of the cells, is performed and a purification section where a purification step, in which the product is purified from the culture supernatant liquid to obtain an active pharmaceutical ingredient of a biopharmaceutical, is performed, to allow the culture supernatant liquid to flow into the purification section from the culture section through the coupling line; connecting a flexible single-use tank having an accommodation capacity of 0.2 times to 10 times an amount of the culture liquid in the culture tank to a branch line provided between the culture section and the purification section; and temporarily storing the culture supernatant liquid in the tank.

It is preferable that all flow passages of the culture supernatant liquid from the culture section to the tank are sterilely connected.

It is preferable that both a connecting portion of the branch line with the tank and a connecting portion of the tank with the branch line are sterile connectors, and the branch line and the tank are sterilely connected to each other through the sterile connectors.

It is preferable that a tube constituting the branch line and a flow-in tube connected to an inlet port of the tank for the culture supernatant liquid are sterilely connected to each other by thermal welding.

It is preferable that the culture supernatant liquid flowing into the purification section is filtered by a sterile filter provided downstream of the coupling line with respect to a connecting portion of the branch line.

It is preferable that the amount of the culture liquid in the culture tank is 50 L or more and 5,000 L or less.

It is preferable that the temporarily stored culture supernatant liquid is discarded from a discharge port provided in the tank.

It is preferable that the temporarily stored culture supernatant liquid is allowed to flow into the purification section from a discharge port provided in the tank.

It is preferable to use a plurality of the branch lines provided between the culture section and the purification section.

It is preferable that, in a case where the tank connected to one of the plurality of the branch lines is filled with the culture supernatant liquid, the tank connected to another branch line is switched for use.

It is preferable that, in a case where a malfunction occurs in the purification section, a flow passage of the culture supernatant liquid is switched from the coupling line to the branch line.

It is preferable that the flow passage of the culture supernatant liquid is switched using a no liquid-contact type valve capable of switching the flow passage without coming into contact with the culture supernatant liquid.

It is preferable that the flow passage of the culture supernatant liquid is switched using the no liquid-contact type valve provided in the branch line and the no liquid-contact type valve provided downstream of the coupling line with respect to a connecting portion of the branch line.

It is preferable that the no liquid-contact type valve is a type in which a tube is sandwiched from an outside.

It is preferable that, in a case where a signal indicating that a malfunction has occurred is output from the purification section, control of switching the flow passage of the culture supernatant liquid from the coupling line to the branch line is performed.

It is preferable to include measuring a flow rate of the culture supernatant liquid with a flowmeter provided upstream of the coupling line with respect to a connecting portion of the branch line; and controlling the flow rate of the culture supernatant liquid based on a measurement result.

It is preferable that the purification section continuously purifies the culture supernatant liquid.

It is preferable that the product is an antibody.

It is preferable that a concentration of the antibody in the culture supernatant liquid is 1.0 g/L or more.

It is preferable that a concentration of the cells in the culture liquid is 8.0 × 10⁷ cells/mL or more.

A system for producing an active pharmaceutical ingredient of a biopharmaceutical according to the present disclosure includes a culture section where a culture step, in which cells are cultured in a culture liquid stored in a culture tank and the cells are removed from the culture liquid to obtain a culture supernatant liquid containing a product of the cells, is performed; a purification section where a purification step, in which the product is purified from the culture supernatant liquid to obtain an active pharmaceutical ingredient of a biopharmaceutical, is performed; a coupling line which connects the culture section and the purification section, through which the culture supernatant liquid flows from the culture section to the purification section; a branch line which is provided between the culture section and the purification section; and a flexible single-use tank which is connected to the branch line, has an accommodation capacity of 0.2 times to 10 times an amount of the culture liquid in the culture tank, and temporarily stores the culture supernatant liquid.

An active pharmaceutical ingredient of a biopharmaceutical according to the present disclosure is produced by the method for producing an active pharmaceutical ingredient of a biopharmaceutical described above.

According to the technology of the present disclosure, it is possible to provide a method for producing an active pharmaceutical ingredient of a biopharmaceutical, which is capable of reducing a concern of contamination and is easy to handle; a system for producing an active pharmaceutical ingredient of a biopharmaceutical; and an active pharmaceutical ingredient of a biopharmaceutical.

### BRIEF DESCRIPTION OF THE DRAWINGS

Fig. 1 is a diagram showing a system for producing an active pharmaceutical ingredient of a biopharmaceutical.
Fig. 2 is a diagram showing a structure of a pinch valve.
Fig. 3 is a block diagram of a computer constituting a control device.
Fig. 4 is a block diagram of a CPU of the computer constituting the control device.
Fig. 5 is a diagram showing processing of an intermediate section control unit in a case where a malfunction occurs in a purification section.
Fig. 6 is a diagram showing a state in which, in a case where one tank is filled with a culture supernatant liquid, the tank is switched to another tank for use.
Fig. 7 is a flowchart for describing an action of the technology of the present disclosure.
Fig. 8 is a diagram showing an aspect in which a tube constituting a branch line and a flow-in tube connected to an inlet port of a tank for the culture supernatant liquid are sterilely connected to each other by thermal welding.
Fig. 9 is a diagram showing details of the thermal welding.
Fig. 10 is a diagram showing a screw-type tube clamp.
Fig. 11 is a diagram showing an aspect in which the culture supernatant liquid temporarily stored in the tank flows into the purification section from a discharge port provided in the tank.
Fig. 12 is a diagram showing an aspect in which the culture supernatant liquid temporarily stored in the tank is discarded or flows into the purification section from a discharge port provided in the tank.
Fig. 13 is a table showing Examples and Comparative Examples.

### DESCRIPTION OF THE PREFERRED EMBODIMENTS

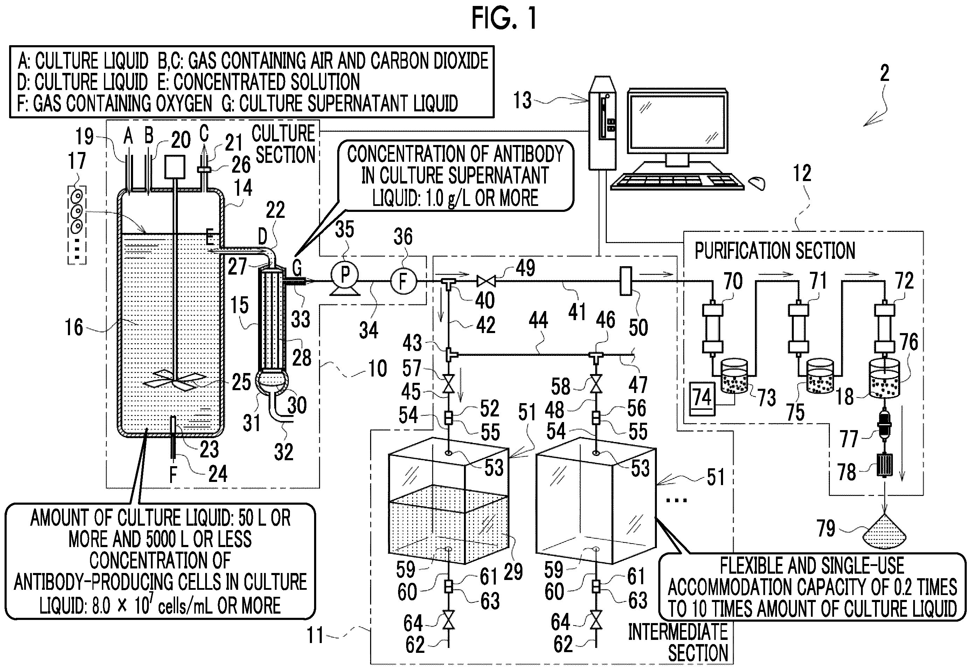

As an example shown in Fig. 1, a system 2 for producing an active pharmaceutical ingredient of a biopharmaceutical according to the technology of the present disclosure, which is used for performing a method for producing an active pharmaceutical ingredient of a biopharmaceutical, includes a culture section 10 where a culture step is performed, an intermediate section 11, a purification section 12 where a purification step is performed, and a control device 13. The culture section 10 includes a culture tank 14 and a filter 15. A culture liquid (medium) 16 is stored in the culture tank 14. An amount of the culture liquid 16 in the culture tank 14 is 50 liters (L) or more and 5,000 L or less. Antibody-producing cells 17 are seeded in the culture tank 14, and are cultured in the culture liquid 16. A concentration of the antibody-producing cells 17 in the culture liquid 16 is 8.0 × 10⁷ cells/mL or more. The "cells" is the number of cells.

The antibody-producing cells 17 are, for example, cells established by incorporating an antibody gene into a cell such as a CHO cell. The antibody-producing cells 17 produce an immunoglobulin, that is, an antibody 18 as a product in the process of culturing. Therefore, not only the antibody-producing cells 17 but also the antibody 18 is present in the culture liquid. The antibody 18 is, for example, a monoclonal antibody, and is an active ingredient of a biopharmaceutical.

The culture tank 14 is provided with a culture liquid supply path 19, a gas supply path 20, an exhaust path 21, a culture liquid delivery and recovery path 22, a sparger 23, a gas supply path 24, a stirring blade 25, and the like. The culture liquid supply path 19 is a flow passage for continuously supplying a fresh culture liquid 16 into the culture tank 14, as indicated by an arrow A. That is, the culture section 10 performs perfusion culture. The gas supply path 20 is a flow passage for supplying a gas containing air and carbon dioxide from the upper part, as indicated by an arrow B. As indicated by an arrow C, the exhaust path 21 is a flow passage for exhausting the gas supplied from the gas supply path 20 to the outside of the culture tank 14. An exhaust filter 26 is provided in the exhaust path 21.

The culture liquid delivery and recovery path 22 is connected to an inlet and outlet 27 of the filter 15. The culture liquid delivery and recovery path 22 is a flow passage for delivering the culture liquid 16 in the culture tank 14 to the filter 15, as indicated by an arrow D. In addition, the culture liquid delivery and recovery path 22 is a flow passage for returning the culture liquid 16 (concentrated solution) from the filter 15 to the culture tank 14, as indicated by an arrow E. Although not shown, a pump which supplies the culture liquid 16 into the culture tank 14 is provided in the culture liquid supply path 19.

The sparger 23 is disposed at the bottom of the culture tank 14. As indicated by an arrow F, the sparger 23 releases gas containing oxygen, which is supplied from the gas supply path 24, to the culture tank 14. The oxygen released from the sparger 23 is dissolved in the culture liquid 16, and supports production activity of the antibody 18 of the antibody-producing cells 17. The stirring blade 25 is rotated at a predetermined rotation speed by a motor or the like to stir the culture liquid 16 in the culture tank 14. As a result, homogeneity of the culture liquid 16 in the culture tank 14 is maintained. In addition to these members, the culture tank 14 is provided with a flow passage for a cell breeding treatment of intentionally taking out a part of the culture liquid 16, for example.

The filter 15 has a filter membrane 28 inside. The filter membrane 28 captures the antibody-producing cells 17 and transmits the antibody 18. With the filter 15, a culture supernatant liquid 29 is obtained by, for example, Tangential Flow Filtration (TFF) method in which the antibody-producing cells 17 are removed from the culture liquid 16 by the filter membrane 28.

More specifically, the filter 15 has a diaphragm pump 31 having an elastic film 30 inside, and a degassing and gas suppling path 32. Air on the lower side of the elastic film 30 is degassed from the degassing and gas suppling path 32, and the elastic film 30 is elastically deformed to be attached to the lower end of the diaphragm pump 31, whereby the culture liquid 16 in the culture tank 14 flows into the filter 15 through the culture liquid delivery and recovery path 22. In addition, the culture liquid 16 (concentrated solution) which cannot pass through the filter membrane 28 is returned to the culture tank 14 through the culture liquid delivery and recovery path 22, by supplying air from the degassing and gas suppling path 32 to the lower side of the elastic film 30 and elastically deforming the elastic film 30 to be attached to the upper side of the diaphragm pump 31.

The culture supernatant liquid 29 flows out from an outlet 33 of the filter 15, as indicated by an arrow G. The culture supernatant liquid 29 mainly contains the antibody 18. The culture supernatant liquid 29 contains, in addition to the antibody 18, contaminants such as a cell-derived protein, cell-derived Deoxyribonucleic Acid (DNA), and an aggregate of the antibody 18, a virus, and the like. A concentration of the antibody 18 in the culture supernatant liquid 29 is 1.0 g/L or more.

A coupling path 34 is connected to an outlet 33 of the filter 15. The coupling path 34 is provided with a delivery pump 35 and a flowmeter 36. The delivery pump 35 sends out the culture supernatant liquid 29 flowed out from the outlet 33 downstream of the intermediate section 11 and the purification section 12. The flowmeter 36 measures a flow rate of the culture supernatant liquid 29 flowing through the coupling path 34.

In the intermediate section 11, the coupling path 34 is sterilely connected to a coupling path 41 through a three-way joint 40. The coupling path 41 is connected to the purification section 12. That is, the coupling paths 34 and 41 couple the culture section 10 and the purification section 12. The coupling paths 34 and 41 are an example of "coupling line" according to the technology of the present disclosure.

The coupling path 34 is also sterilely connected to a branch path 42 through the three-way joint 40. The branch path 42 is sterilely connected to branch paths 44 and 45 through a three-way joint 43. In addition, a branch path 44 is sterilely connected to branch paths 47 and 48 through a three-way joint 46. Although not shown, a plurality of the branch paths are also sterilely connected beyond the branch path 47. These branch paths 42, 44, 45, 47, 48, and the like are an example of "branch line" according to the technology of the present disclosure. That is, a plurality of the branch lines are provided between the culture section 10 and the purification section 12. In addition, the three-way joint 40 is an example of "connecting portion" in "downstream of the coupling line with respect to a connecting portion of the branch line" according to the technology of the present disclosure.

The coupling path 41 is provided with a pinch valve 49 and a sterile filter 50. The pinch valve 49 and the sterile filter 50 are provided downstream of the three-way joint 40, which is the connecting portion with the branch path 42, in the coupling path 41. The pinch valve 49 is a valve for switching a flow passage of the culture supernatant liquid 29 from the coupling path 41 to the branch path 42. The pinch valve 49 is an example of "no liquid-contact type valve" according to the technology of the present disclosure. The sterile filter 50 filters the culture supernatant liquid 29 flowing into the purification section 12. The sterile filter 50 includes, for example, a filter membrane having a pore diameter of 0.2 µm.

A tank 51 is connected to the branch paths 45 and 48. The tank 51 temporarily stores the culture supernatant liquid 29 from the culture section 10 which flows in from the branch path 42 and the like. The tank 51 is formed of, for example, a resin film such as polyolefin (PO) and ethylene-vinyl acetate (EVA) copolymer in a rectangular shape, and has flexibility. In addition, the tank 51 is made of a single-use material which can be used only once.

The tank 51 has an accommodation capacity of 0.2 times to 10 times the amount of the culture liquid 16 in the culture tank 14. Therefore, for example, in a case where the amount of the culture liquid 16 is 50 L, which is the lower limit value, the accommodation capacity of the tank 51 is 10 L to 500 L. In addition, for example, in a case where the amount of the culture liquid 16 is 5,000 L, which is the upper limit value, the accommodation capacity of the tank 51 is 1,000 L to 50,000 L. The "0.2 times to 10 times" means 0.2 times or more and 10 times or less. It should be noted that the tank 51 need not always be connected to the branch line, as in the branch paths 45 and 48. The accommodation capacity of the tank 51 is preferably 1 times to 5 times the amount of the culture liquid 16 in the culture tank 14, and more preferably 1.5 times to 5 times the amount of the culture liquid 16 in the culture tank 14.

A sterile connector 52 is provided at a distal end of the branch path 45. In addition, a sterile connector 55 is also provided at a distal end of a flow-in tube 54 connected to an inlet port 53 of the tank 51 for the culture supernatant liquid 29. Through the sterile connectors 52 and 55, the branch path 45 is sterilely connected to the flow-in tube 54, and thus sterilely connected to the tank 51. Similarly, a sterile connector 56 is also provided at a distal end of the branch path 48, and the branch path 48 and the tank 51 are sterilely connected to each other through the sterile connectors 55 and 56. The flow passage of the culture supernatant liquid 29 from the culture section 10 to the tank 51 is all sterilely connected by the sterile connectors 52, 55, and 56, the three-way joints 40, 43, and 46, and the like. The distal end of the branch path 45 and the distal end of the branch path 48 are an example of "connecting portion of the branch line with the tank" according to the technology of the present disclosure. In addition, the distal end of the flow-in tube 54 is an example of "connecting portion of the tank with the branch line" according to the technology of the present disclosure. As the sterile connector 52 and the like, for example, ReadyMate^{™} Disposable Aseptic Connector (DAC) manufactured by Global Life Science Technologies Japan, Inc. can be used.

The branch path 45 is provided with a pinch valve 57. In addition, the branch path 48 is provided with a pinch valve 58. The pinch valve 57 is a valve for allowing the culture supernatant liquid 29 to flow into the tank 51 through the branch path 45. The pinch valve 58 is a valve for allowing the culture supernatant liquid 29 to flow into the tank 51 through the branch path 48. Similarly to the pinch valve 49, the pinch valves 57 and 58 are an example of "no liquid-contact type valve" according to the technology of the present disclosure.

A liquid level detection sensor (not shown) is provided in the tank 51. In a case where the liquid level detection sensor detects that the culture supernatant liquid 29 has reached a predetermined water level, a signal indicating that the tank 51 is filled with the culture supernatant liquid 29 is transmitted to the control device 13.

A discharge port 59 is provided at the bottom of the tank 51. A discharge tube 60 is connected to the discharge port 59. A sterile connector 61 is provided at a distal end of the discharge tube 60. A waste liquid path 62 is connected to the discharge tube 60. A sterile connector 63 is provided at a distal end of the waste liquid path 62. Through the sterile connectors 61 and 63, the waste liquid path 62 is sterilely connected to the discharge tube 60, and thus sterilely connected to the tank 51.

A manual pinch valve 64 is provided in the waste liquid path 62. In a case where the pinch valve 64 is opened, the culture supernatant liquid 29 temporarily stored in the tank 51 is discarded as waste liquid to a waste liquid tank (not shown) through the discharge port 59, the discharge tube 60, and the waste liquid path 62. The reason why the culture supernatant liquid 29 from the culture section 10 is once stored in the tank 51 and then discarded into the waste liquid path 62, without being directly discarded into the waste liquid path 62, is to prevent contamination from the waste liquid path 62. Although not shown, a sterile filter may be installed between the waste liquid path 62 and the waste liquid tank.

The purification section 12 includes an immunaffinity chromatography device 70, a cation chromatography device 71, and an anion chromatography device 72, and continuously purifies the culture supernatant liquid 29 by these chromatography devices 70 to 72. The immunaffinity chromatography device 70 is allowed to flow the culture supernatant liquid 29 thereto through the coupling path 41. The immunaffinity chromatography device 70 extracts the antibody 18 from the culture supernatant liquid 29 using a column in which a ligand such as protein A and protein G, having an affinity for the antibody 18, is immobilized on a carrier, thereby generating a first purified liquid 73. The first purified liquid 73 is subjected to a treatment 74 for inactivating the virus (hereinafter, referred to as a virus inactivating treatment).

The first purified liquid 73 after the virus inactivating treatment 74 is performed flows into the cation chromatography device 71. The cation chromatography device 71 extracts the antibody 18 from the first purified liquid 73 using a column in which a cation exchanger is used as a stationary phase, thereby generating a second purified liquid 75. The second purified liquid 75 flows into the anion chromatography device 72. The anion chromatography device 72 extracts the antibody 18 from the second purified liquid 75 using a column in which an anion exchanger is used as a stationary phase, thereby generating a third purified liquid 76. The third purified liquid 76 is passed through a filter 77 to remove the virus. Thereafter, the third purified liquid 76 is subjected to a concentration and filtration treatment by Ultrafiltration (UF) and Diafiltration (DF) through a filter 78. As a result, an active pharmaceutical ingredient 79 of a biopharmaceutical is obtained. By sequentially performing the component separation treatment by such a plurality of types of chromatography devices 70 to 72, the contaminants and the virus are removed from the culture supernatant liquid 29 in a stepwise manner, and the purity of the antibody 18 is increased in a stepwise manner. A single-pass TFF-type filter may be provided in front of the immunaffinity chromatography device 70.

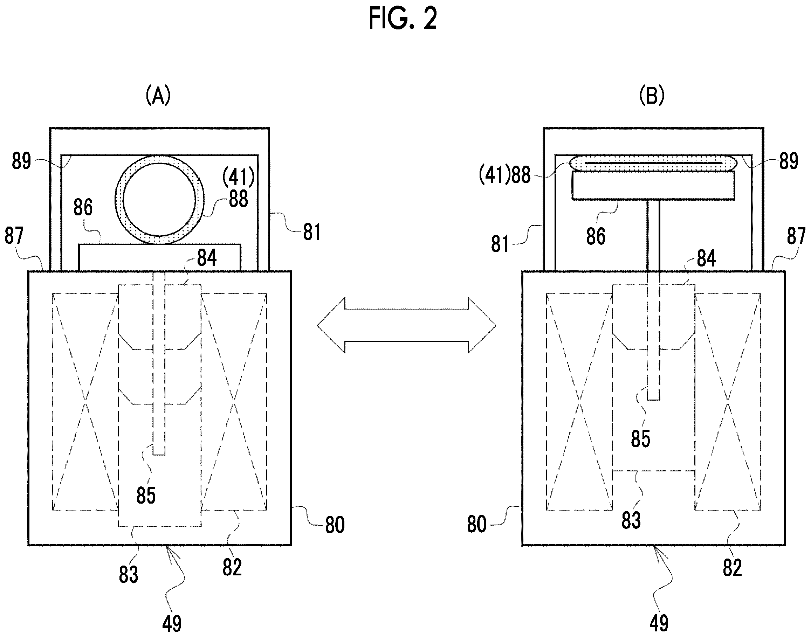

As an example shown in Fig. 2, the pinch valve 49 includes a body part 80 and a holder 81. A cylindrical coil 82, a movable iron core 83, a fixed iron core 84, and a shaft 85 are provided in the body part 80. The movable iron core 83 is accommodated in a lower part of the coil 82 in the cylindrical space. The fixed iron core 84 is fixed to an upper end of the coil 82 in the cylindrical space. A lower end of the shaft 85 is attached to the movable iron core 83. A rectangular valve body 86 disposed in the holder 81 is attached to an upper end of the shaft 85.

The holder 81 is a substantially U-shaped member attached to an upper end surface 87 of the body part 80. A tube 88 constituting the coupling path 41 is inserted into the holder 81. The tube 88 is held in a state of being sandwiched between the valve body 86 and an action surface 89 of the holder 81, facing the upper end surface 87 of the body part 80.

In a case where current is not flowed through the coil 82, as shown in (A), the movable iron core 83 is located at the lower end of the body part 80, and thus the valve body 86 is located on the upper end surface 87 side of the body part 80. In this case, the tube 88 is not flattened by the valve body 86, and thus the coupling path 41 is open. Therefore, in the case of (A), the culture supernatant liquid 29 flows from the culture section 10 to the purification section 12 through the coupling path 41. On the other hand, in a case where current is flowed through the coil 82, as shown in (B), the movable iron core 83 is attracted to the fixed iron core 84 by the action of the magnetic field generated in the coil 82, and thus the valve body 86 moves toward the side of the action surface 89. The tube 88 is flattened by the valve body 86 and the action surface 89, and thus the coupling path 41 is blocked. Therefore, in the case of (B), the flow of the culture supernatant liquid 29 from the culture section 10 to the purification section 12 through the coupling path 41 is blocked. The fixed iron core 84 also serves as a stopper which restricts the movement of the movable iron core 83, and thus the movement of the valve body 86 upward.

In this way, the pinch valve 49 is a method of pinching the tube 88 from the outside, and the flow passage can be switched without contacting the culture supernatant liquid 29. Since the other pinch valves 57 and 58 also have the same configuration and the same action as the pinch valve 49, except that the tube inserted into the holder 81 is different, the detailed description thereof will be omitted. In addition, since the pinch valve 64 has the same configuration and the same operation as the pinch valve 49, except that the tube inserted into the holder 81 is different and the pinch valve 64 is manually operated, the detailed description thereof will be omitted.

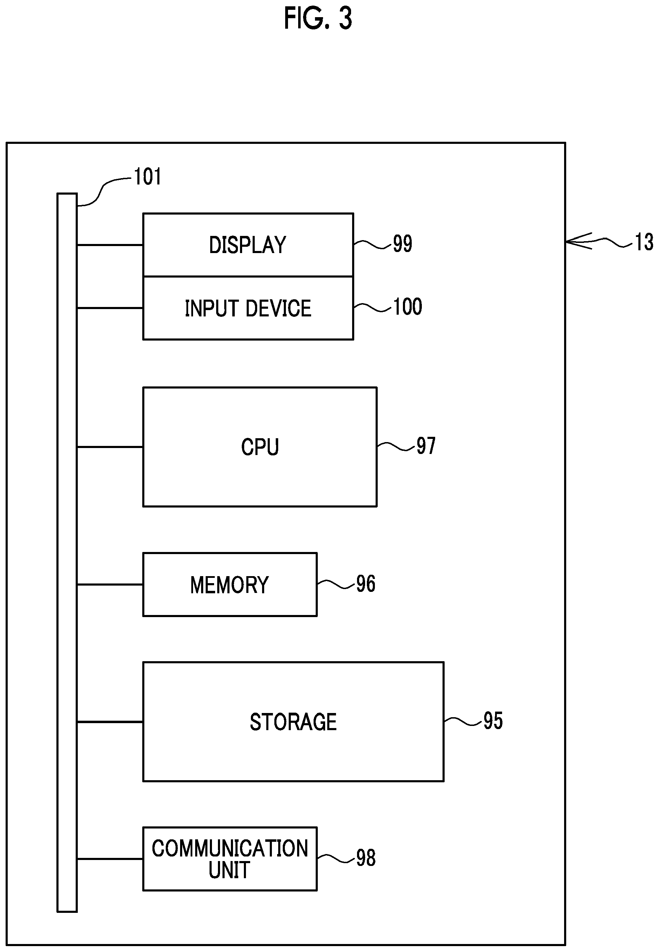

As an example shown in Fig. 3, a computer constituting the control device 13 includes a storage 95, a memory 96, a central processing unit (CPU) 97, a communication unit 98, a display 99, and an input device 100. These units are connected to one another through a bus line 101.

The storage 95 is a hard disk drive that is provided in the computer constituting the control device 13 or is connected to the computer through a cable or a network. Alternatively, the storage 95 is a disk array in which a plurality of hard disk drives are connected. The storage 95 stores, for example, a control program, such as an operating system, various application programs, and various types of data associated with these programs. In addition, a solid state drive may be used instead of the hard disk drive.

The memory 96 is a work memory for the CPU 97 to perform processes. The CPU 97 loads the program stored in the storage 95 to the memory 96 and performs a process corresponding to the program. As a result, the CPU 97 integrally controls each unit of the computer. The memory 96 may be incorporated in the CPU 97.

The communication unit 98 controls transmission of various information to an external apparatus. The display 99 displays various screens. The various screens have operation functions by a graphical user interface (GUI). The computer constituting the control device 13 receives operation instructions input from the input device 100 through various screens. The input device 100 is, for example, a keyboard, a mouse, a touch panel, and a microphone for voice input.

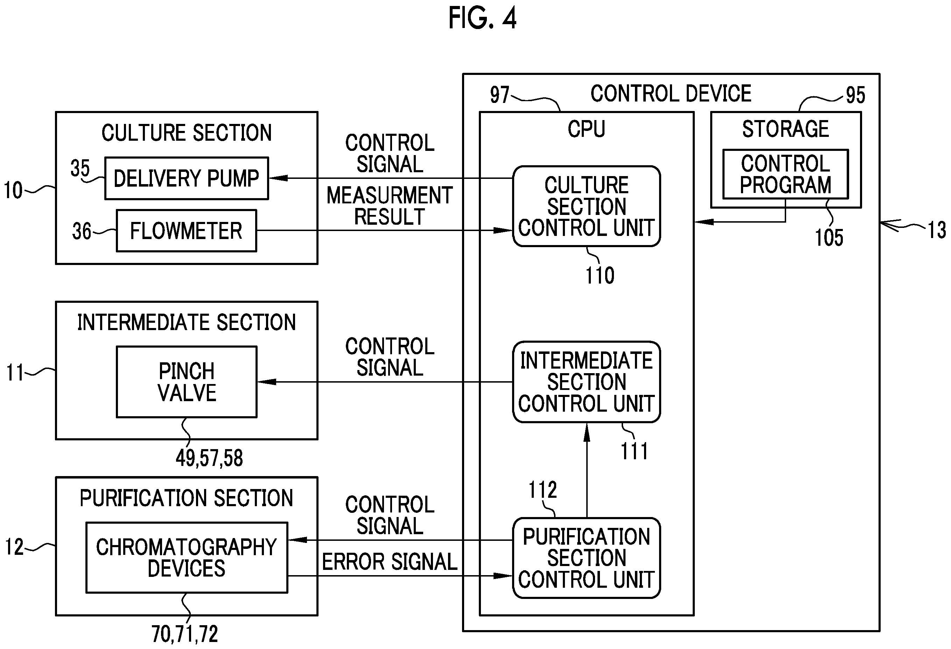

As an example shown in Fig. 4, a control program 105 is stored in the storage 95 of the control device 13. The control program 105 is an application program for causing the computer to function as the control device 13. In addition, the storage 95 stores data of various types of screens to be displayed on the display 99.

In a case where the control program 105 is activated, the CPU 97 of the computer constituting the control device 13 functions as a culture section control unit 110, an intermediate section control unit 111, and a purification section control unit 112, in cooperation with the memory 96 and the like. The culture section control unit 110 controls the operation of the culture section 10, the intermediate section control unit 111 controls the operation of the intermediate section 11, and the purification section control unit 112 controls the operation of the purification section 12.

The culture section control unit 110 receives the measurement result of the flow rate of the culture supernatant liquid 29 from the flowmeter 36. The culture section control unit 110 controls the flow rate of the culture supernatant liquid 29 based on the measurement result. More specifically, the culture section control unit 110 transmits, to the delivery pump 35, a control signal for setting the flow rate of the culture supernatant liquid 29 to a preset value. In addition, the culture section control unit 110 also controls the supply amount of the culture liquid 16 through the culture liquid supply path 19, the supply amount of the gas containing oxygen through the sparger 23 and the gas supply path 24, the rotation speed of the stirring blade 25, and the like.

The intermediate section control unit 111 transmits a control signal to the pinch valves 49, 57, and 58. The control signal is a signal for transmitting any one of an instruction not to flow the current to the coil 82 in the pinch valve 49 or the like, or an instruction to flow the current to the coil 82. In a case where the control signal for transmitting the instruction not to flow the current to the coil 82 is received, the pinch valve 49 or the like opens the flow passage as shown in (A) of Fig. 2. On the other hand, in a case where the control signal for transmitting the instruction to flow the current to the coil 82 is received, the pinch valve 49 or the like closes the flow passage as shown in (B) of Fig. 2. Hereinafter, the control signal for transmitting the instruction not to flow the current to the coil 82 is denoted by a control signal (open). In addition, the control signal for transmitting the instruction to flow the current to the coil 82 is denoted by a control signal (close).

The purification section control unit 112 transmits a control signal to the chromatography devices 70 to 72. The chromatography devices 70 to 72 operate the built-in pump, valve, and the like in response to the control signal. In addition, the purification section control unit 112 receives an error signal from the chromatography devices 70 to 72. The error signal is issued from the chromatography devices 70 to 72 in a case where some malfunction occurs in the chromatography devices 70 to 72. The malfunction is, for example, clogging of the column in the chromatography devices 70 to 72, a failure of the pump in the chromatography devices 70 to 72, or the like. The purification section control unit 112 transmits the error signal to the intermediate section control unit 111. The error signal is an example of "signal indicating that a malfunction has occurred" according to the technology of the present disclosure. Although not shown, in a case where the malfunction is resolved, a notification signal indicating the fact is issued from the chromatography devices 70 to 72 toward the purification section control unit 112.

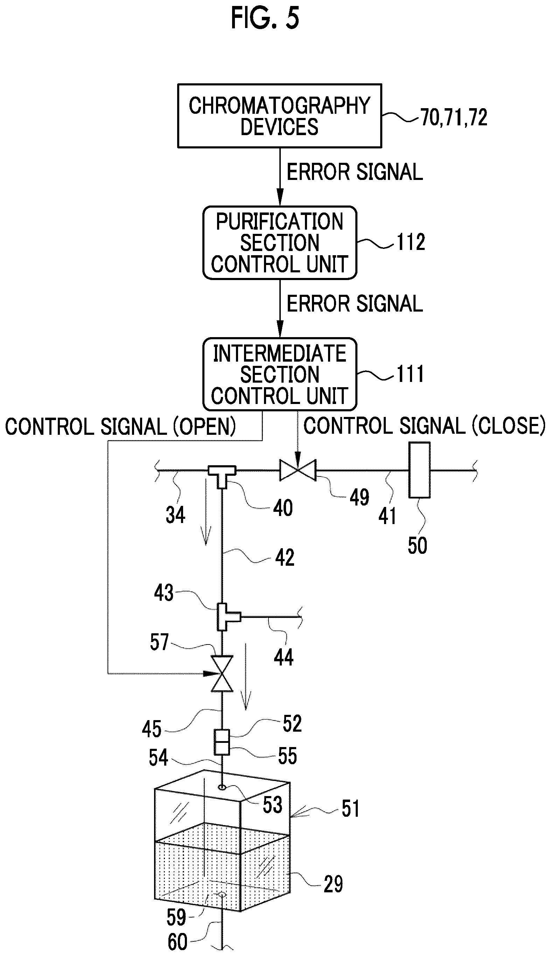

As an example shown in Fig. 5, in a case where the error signal is received from the purification section control unit 112, the intermediate section control unit 111 transmits the control signal (close) to the pinch valve 49 and then transmits the control signal (open) to the pinch valve 57. As a result, the flow passage of the culture supernatant liquid 29 is switched from the coupling path 41 to the branch paths 42 and 45. That is, the intermediate section control unit 111 performs control of switching the flow passage of the culture supernatant liquid 29 from the coupling path 41 to the branch paths 42 and 45 in a case where the error signal indicating that the malfunction has occurred in the purification section 12 is output. The culture supernatant liquid 29 flows into the tank 51 through the branch paths 42 and 45, the flow-in tube 54, and the inlet port 53. Although not shown, in a case where the malfunction in the purification section 12 is resolved and the notification signal indicating the fact is received from the chromatography devices 70 to 72, the intermediate section control unit 111 transmits the control signal (close) to the pinch valve 57 and then transmits the control signal (open) to the pinch valve 49, and performs control of returning the flow passage of the culture supernatant liquid 29 from the branch paths 42 and 45 to the coupling path 41.

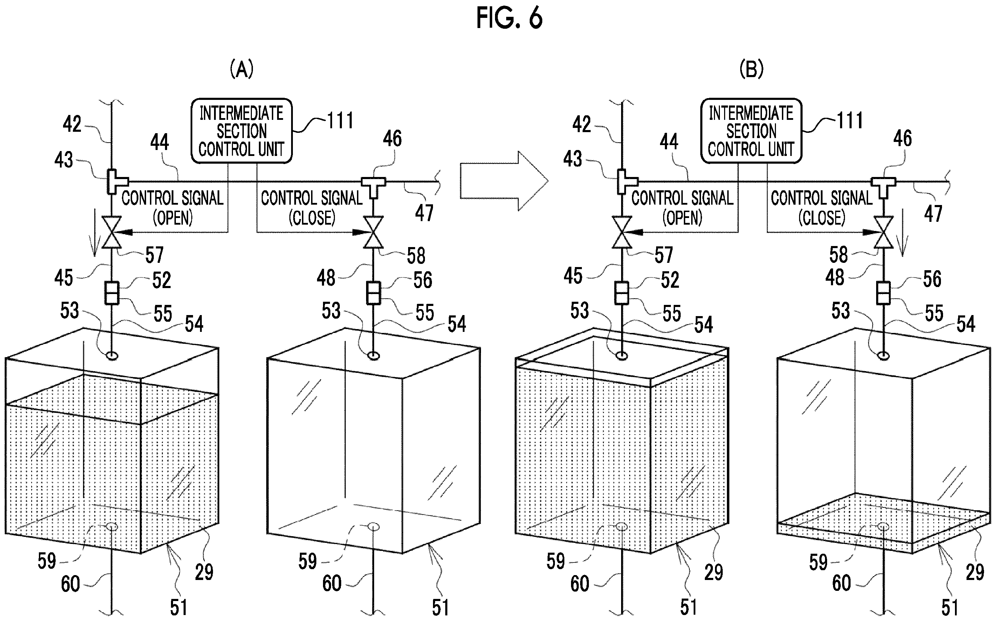

As an example shown in Fig. 6, (A) shows a state in which the intermediate section control unit 111 transmits the control signal (open) to the pinch valve 57 of the branch path 45 and transmits the control signal (close) to the pinch valve 58 of the branch path 48, respectively, and the culture supernatant liquid 29 is temporarily stored in the tank 51 connected to the branch path 45. In the state shown in (A), in a case where a signal indicating that the tank 51 connected to the branch path 45 is filled with the culture supernatant liquid 29 is received from the liquid level detection sensor of the tank 51, as shown in (B), the intermediate section control unit 111 transmits the control signal (close) to the pinch valve 57 of the branch path 45 and then transmits the control signal (open) to the pinch valve 58 of the branch path 48. As a result, the flow passage of the culture supernatant liquid 29 is switched from the branch path 45 to the branch paths 44 and 48, and the culture supernatant liquid 29 flows into the tank 51 to which the branch path 48 is connected. That is, in a case where the tank 51 connected to the branch path 45, which is one of the plurality of branch lines, is filled with the culture supernatant liquid 29, the intermediate section control unit 111 switches the use to the tank 51 connected to the branch path 48, which is another branch line. Although not shown, in a case where the tank 51 connected to the branch path 48 is filled with the culture supernatant liquid 29, the intermediate section control unit 111 switches the use to the tank 51 connected to the branch path 45 or other branch paths prior to the branch path 47.

The tank 51 connected to the branch path 45, filled with the culture supernatant liquid 29, is separated from the branch path 45 by manually opening the pinch valve 64 to discard the culture supernatant liquid 29 and then detaching the sterile connector 55 from the sterile connector 52. A sterile connector 55 of a new empty tank 51 is attached to the sterile connector 52. As a result, the used tank 51 is exchanged for the new empty tank 51.

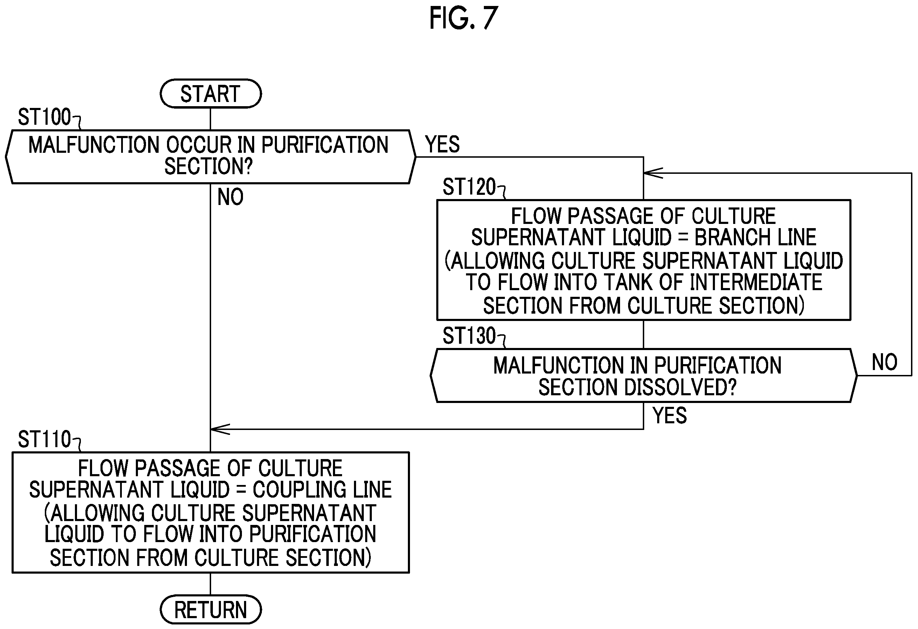

Next, an operation according to the above-described configuration will be described with reference to a flowchart illustrated in Fig. 7. First, the antibody-producing cells 17 are seeded in the culture tank 14 of the culture section 10, and are cultured in the culture liquid 16. As a result, the antibody 18 is produced from the antibody-producing cells 17, and is dispersed in the culture liquid 16.

The culture liquid 16 is sent to the filter 15 through the culture liquid delivery and recovery path 22 and the inlet and outlet 27. The antibody-producing cells 17 are removed from the culture liquid 16 by the filter membrane 28 of the filter 15, and the culture supernatant liquid 29 is obtained. The culture supernatant liquid 29 flows out from the outlet 33 to the coupling path 34, and is sent downstream by the delivery pump 35. In this case, the control signal according to the measurement result of the flowmeter 36 is transmitted from the culture section control unit 110 to the delivery pump 35, whereby the flow rate of the culture supernatant liquid 29 is controlled based on the measurement result of the flowmeter 36.

In a case where no malfunction occurs in the purification section 12 (NO in the step ST100), the intermediate section control unit 111 transmits the control signal (open) to the pinch valve 49 and the control signal (close) to the pinch valves 57 and 58. As a result, the coupling path 41 is opened, and the branch path 45 and the like are closed. In this way, the culture supernatant liquid 29 flows from the coupling path 34 to the coupling path 41. The culture supernatant liquid 29 which has flowed into the coupling path 41 is filtered by the sterile filter 50, and then flows into the purification section 12 (step ST110). In the purification section 12, the antibody 18 in the culture supernatant liquid 29 is continuously purified by the chromatography devices 70 to 72, and finally the active pharmaceutical ingredient 79 of a biopharmaceutical is obtained.

On the other hand, in a case where the malfunction occurs in the purification section 12 (YES in the step ST100), the error signal is issued from the chromatography devices 70 to 72 to the purification section control unit 112. The error signal is transmitted from the purification section control unit 112 to the intermediate section control unit 111. In this case, as shown in Fig. 5, from the intermediate section control unit 111, the control signal (close) is transmitted to the pinch valve 49, and then the control signal (open) is transmitted to the pinch valve 57. As a result, the coupling path 41 is closed, and the branch path 45 is opened. In this way, the culture supernatant liquid 29 flows into the tank 51 through the branch paths 42 and 45, the flow-in tube 54, and the inlet port 53 (step ST120). The state in which the culture supernatant liquid 29 flows into the tank 51 and then temporarily stored is continued until the malfunction in the purification section 12 is resolved (NO in the step ST130).

As shown in Fig. 6, in a case where the tank 51 connected to the branch path 45 is filled with the culture supernatant liquid 29, from the intermediate section control unit 111, the control signal (close) is transmitted to the pinch valve 57 of the branch path 45, and then the control signal (open) is transmitted to the pinch valve 58 of the branch path 48. As a result, the flow passage of the culture supernatant liquid 29 is switched from the branch path 45 to the branch paths 44 and 48, and the culture supernatant liquid 29 flows into the tank 51 to which the branch path 48 is connected.

In a case where the malfunction in the purification section 12 is resolved (YES in the step ST130), from the intermediate section control unit 111, the control signal (close) is transmitted to the pinch valve 57 or 58, and then the control signal (open) is transmitted to the pinch valve 49. As a result, the coupling path 41 is opened again, the branch path 45 or 48 is closed, and the culture supernatant liquid 29 flows from the coupling path 34 to the coupling path 41.

As described above, in the method for producing an active pharmaceutical ingredient of a biopharmaceutical according to the technology of the present disclosure, the culture section 10 where the culture step, in which the antibody-producing cells 17 are cultured in the culture liquid 16 stored in the culture tank 14 and the antibody-producing cells 17 are removed from the culture liquid 16 to obtain the culture supernatant liquid 29 containing the antibody 18, is performed is connected, through the coupling paths 34 and 41, to the purification section 12 where the purification step, in which the antibody 18 is purified from the culture supernatant liquid 29 to obtain the active pharmaceutical ingredient 79 of a biopharmaceutical, is performed, and the culture supernatant liquid 29 is allowed to flow into the purification section 12 from the culture section 10 through the coupling paths 34 and 41. Then, the flexible single-use tank 51 having an accommodation capacity of 0.2 times to 10 times the amount of the culture liquid 16 in the culture tank 14 is connected to the branch paths 45 and 48 provided between the culture section 10 and the purification section 12, and the culture supernatant liquid 29 is temporarily stored in the tank 51.

Since the tank 51 has flexibility, the tank 51 can be folded compactly in a non-use state, and is easier to handle than the glass tank disclosed in JP2017-515501A. In addition, since the tank 51 is made of a single-use material and is not repeatedly used as the glass tank disclosed in JP2017-515501A, there is little concern about contamination of the culture supernatant liquid 29. Furthermore, since the tank 51 is made of a single-use material, sterilization is not required as the glass tank disclosed in JP2017-515501A. In addition, it is easier to discard than the glass tank disclosed in JP2017-515501A.

The tank 51 has an accommodation capacity of 0.2 times to 10 times the amount of the culture liquid 16 in the culture tank 14. Here, in a case where the tank 51 has an accommodation capacity equal to or less than 0.2 times the amount of the culture liquid 16 in the culture tank 14, since the tank 51 is immediately filled with the culture supernatant liquid 29, it is necessary to frequently discard the waste liquid, or in a case where the discharge port 59 is not provided in the tank 51, it is necessary to frequently replace the tank 51, which are inefficient. On the contrary, in a case where the tank 51 has an accommodation capacity larger than 10 times the amount of the culture liquid 16 in the culture tank 14, the tank 51 is too large to handle. Therefore, the tank 51 having an accommodation capacity of 0.2 times to 10 times the amount of the culture liquid 16 in the culture tank 14 is an appropriate size for temporarily storing the culture supernatant liquid 29. Therefore, the method for producing an active pharmaceutical ingredient of a biopharmaceutical according to the technology of the present disclosure can reduce the concern of contamination and is easy to handle. In addition, the active pharmaceutical ingredient 79 of a biopharmaceutical, which is produced by the method for producing an active pharmaceutical ingredient of a biopharmaceutical according to the technology of the present disclosure, is less likely to be affected by the contamination, and is easily produced. As described above, the accommodation capacity of the tank 51 is preferably 1 times to 5 times the amount of the culture liquid 16 in the culture tank 14, and more preferably 1.5 times to 5 times the amount of the culture liquid 16 in the culture tank 14.

All flow passages of the culture supernatant liquid 29 from the culture section 10 to the tank 51 are sterilely connected. Therefore, it is possible to further reduce the concern of contamination of the culture supernatant liquid 29.

Both the connecting portion between the tank 51 and the branch paths 45 and 48 and the connecting portion between the tank 51 and the branch paths 45 and 48 are the sterile connectors 52, 55, and 56, and thus the branch paths 45 and 48, and the tank 51 are sterilely connected to each other through the sterile connectors 52, 55, and 56. Therefore, it is possible to further reduce the concern of contamination of the culture supernatant liquid 29.

The culture supernatant liquid 29 flowing into the purification section 12 is filtered by the sterile filter 50 provided downstream of the three-way joint 40, which is a connecting portion with the branch path 42, in the coupling path 41. Therefore, it is possible to prevent the invasion of bacteria or the like from the purification section 12 to the intermediate section 11 and the culture section 10, and to maintain the sterile state on the upstream side of the sterile filter 50 including the branch line 42 and the like and the tank 51. Accordingly, it is possible to further reduce the concern of contamination of the culture supernatant liquid 29. The sterile filter 50 is not particularly limited as long as it does not contain living bacteria or other microorganisms and suppresses the permeation of the living bacteria or other microorganisms to the upstream side thereof; and a pore diameter of the filter membrane is preferably 0.001 µm to 0.5 µm, more preferably 0.02 µm to 0.3 µm, and particularly preferably 0.1 µm to 0.3 µm.

The amount of the culture liquid 16 in the culture tank 14 is 50 L or more and 5,000 L or less. In a case where the amount of the culture liquid 16 in the culture tank 14 is within the range, as described above, the accommodation capacity of the tank 51 is 10 L to 500 L, or 1,000 L to 50,000 L. In a case where the tank 51 of such a size is made of glass, substantial cost is required, and sterilization is also time-consuming. Therefore, in a case where the amount of the culture liquid 16 in the culture tank 14 is 50 L or more and 5,000 L or less, it is possible to further exhibit the effect that the concern about contamination can be reduced and the handling is easy. The amount of the culture liquid 16 in the culture tank 14 is more preferably 200 L or more and 5,000 L or less, and particularly preferably 200 L or more and 3,000 L or less.

Quality of the antibody 18 in the culture supernatant liquid 29 temporarily stored in the tank 51 deteriorates with the passage of time. Therefore, the culture supernatant liquid 29 temporarily stored in the tank 51 is discarded from the discharge port 59. In this manner, there is no risk that the culture supernatant liquid 29 in which the quality of the antibody 18 has deteriorated is mistakenly allowed to flow into the purification section 12. In addition, it is possible to reliably prevent the culture supernatant liquid 29 from overflowing from the tank 51. Furthermore, work efficiency is better than in a case where the discharge port 59 is not provided in the tank 51 and the tank 51 is filled with the culture supernatant liquid 29, and it is required to exchange the tank 51 for a new empty tank 51.

As the branch paths 45 and 48, a plurality of branch lines are provided between the culture section 10 and the purification section 12. Therefore, in a case where the tank 51 is connected to each of the plurality of branch lines, for example, a frequency of exchanging the tank 51 can be reduced as compared with a case where one tank 51 is connected to one branch line, and the handling is easy. The number of branch lines is more preferably 2 or more, and particularly preferably 2 or more and 10 or less.

In a case where the tank 51 connected to one of the branch paths 45, 48, and the like is filled with the culture supernatant liquid 29, the tank 51 connected to another branch line is switched for use. Therefore, it is possible to reliably prevent the culture supernatant liquid 29 from overflowing from the tank 51.

In a case where the malfunction occurs in the purification section 12, the flow passage of the culture supernatant liquid 29 is switched from the coupling path 41 to the branch path 42 or the like. Therefore, it is possible to avoid a situation in which the production efficiency is significantly reduced, such as the operation of the culture section 10 being stopped due to the influence of the malfunction in the purification section 12.

The flow passage of the culture supernatant liquid is switched using the pinch valves 49, 57, and 58, which are no liquid-contact type valves capable of switching the flow passage without coming into contact with the culture supernatant liquid 29. Therefore, it is possible to further reduce the concern of contamination of the culture supernatant liquid 29.

The flow passage of the culture supernatant liquid 29 is switched using the pinch valves 57 and 58 provided in the branch paths 45 and 48, and the pinch valve 49 provided downstream of the three-way joint 40, which is a connecting portion with the branch path 42, in the coupling path 41. Therefore, the flow passage of the culture supernatant liquid 29 can be reliably switched to the coupling path 41, the branch path 42, or the like.

The pinch valves 49, 57, and 58 are of a type in which the tube 88 and the like constituting the coupling path 41 are interposed from the outside. Therefore, it is possible to reduce the concern of contamination of the culture supernatant liquid 29 with a simple configuration.

In a case where the error signal indicating that the malfunction has occurred in the purification section 12 is output, the intermediate section control unit 111 performs the control of switching the flow passage of the culture supernatant liquid 29 from the coupling path 41 to the branch path 42 or the like. Therefore, it is possible to save the labor of manually switching the flow passage of the culture supernatant liquid 29 from the coupling path 41 to the branch path 42 or the like.

The flow rate of the culture supernatant liquid 29 is measured by the flowmeter 36 provided upstream of the three-way joint 40, which is the connecting portion of the branch path 42, in the coupling path 34, and the flow rate of the culture supernatant liquid 29 is controlled based on the measurement result. Therefore, the flow rate of the culture supernatant liquid 29 flowing through the coupling path 41 and the like can be kept constant.

The purification section 12 continuously purifies the culture supernatant liquid 29. Therefore, the active pharmaceutical ingredient 79 of a biopharmaceutical can be obtained more efficiently.

The biopharmaceutical containing the antibody 18 as an active ingredient is called an antibody pharmaceutical, and is widely used for the treatment of rare diseases such as hemophilia and Crohn's disease, as well as the treatment of chronic diseases such as cancer, diabetes, and rheumatoid arthritis. Therefore, according to the present example in which the product is the antibody 18, it is possible to contribute to the development of antibody pharmaceutical which is widely used for the treatment of various diseases.

A concentration of the antibody 18 in the culture supernatant liquid 29 is 1.0 g/L or more. Therefore, the active pharmaceutical ingredient 79 of a biopharmaceutical can be obtained more efficiently. The concentration of the antibody 18 in the culture supernatant liquid 29 is more preferably 1.2 g/L or more and 15.0 g/L or less, still more preferably 1.3 g/L or more and 13.0 g/L or less, and particularly preferably 1.4 g/L or more and 10.0 g/L or less.

A concentration of the antibody-producing cells 17 in the culture liquid 16 is 8.0 × 10⁷ cells/mL or more. In a case where the concentration of the antibody-producing cells 17 in the culture liquid 16 is 8.0 × 10⁷ cells/mL or more, the antibody-producing cells 17 have a high probability of dying in a case where the operation of the culture section 10 is stopped. Therefore, it is possible to exhibit an effect that it is possible to avoid a situation in which the production efficiency is significantly reduced, such as the operation of the culture section 10 being stopped due to the influence of the malfunction in the purification section 12. The concentration of the antibody-producing cells 17 in the culture liquid 16 is preferably 8.0 × 10⁷ cells/mL or more and 5.0 × 10⁸ cells/mL or less, more preferably 1.0 × 10⁸ cells/mL or more and 4.0 × 10⁸ cells/mL or less, and particularly preferably 1.0 × 10⁸ cells/mL or more and 3.0 × 10⁸ cells/mL or less.

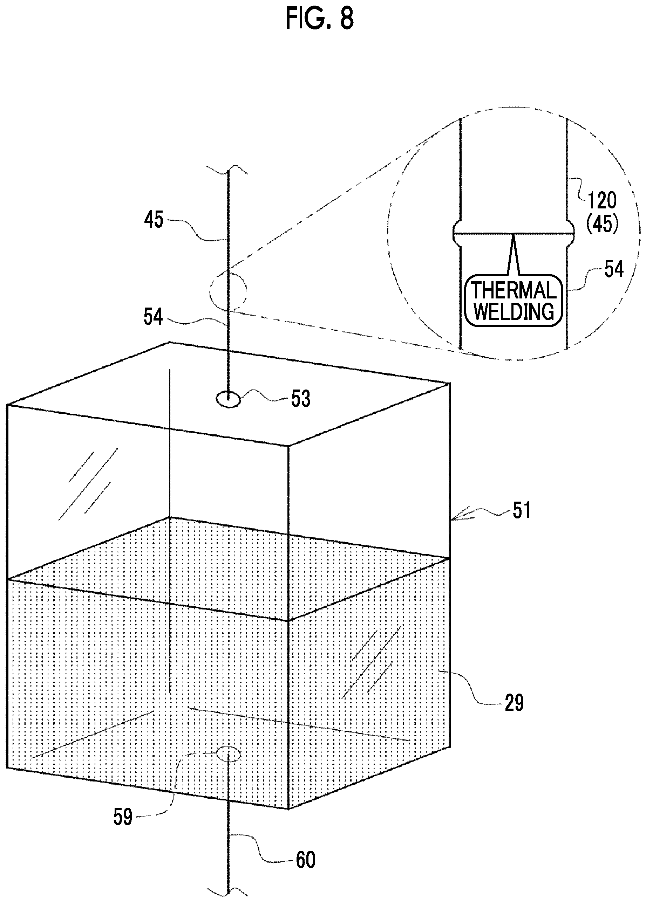

It has been described that the sterile connectors 52, 55, and 56 are as a means for the sterile connection between the branch line and the tank 51, but the present invention is not limited thereto. As an example shown in Fig. 8, for example, a tube 120 constituting the branch path 45 and the flow-in tube 54 may be sterilely connected to each other by thermal welding.

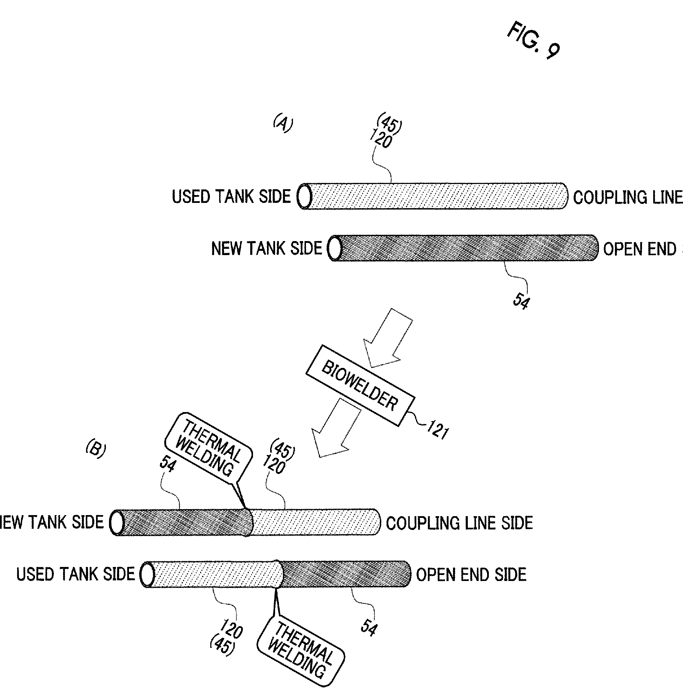

As the thermal welding, for example, Biowelder 121 (Internet Uniform Resource Locator (URL): https://premium.ipros.jp/sartorius-stedim/product/detail/2000608451/?hub=163&categoryId= 52772; see Fig. 9) manufactured by Sartorius Stedim Japan Co., Ltd. is used. In a case where the tank 51 filled with the culture supernatant liquid 29 is exchanged with a new empty tank 51, as an example shown in (A) of Fig. 9, the tube 120 constituting the branch path 45 connected to the tank 51 filled with the culture supernatant liquid 29 (referred to as "used tank" in Fig. 9) and the flow-in tube 54 of the new empty tank 51 (referred to as "new tank" in Fig. 9) are set in a biowelder 121, and the thermal welding is instructed. As a result, as shown in (B), the coupling line side of the tube 120 constituting the branch path 45 and the tank 51 side of the flow-in tube 54 of the new empty tank 51 are sterilely connected to each other by the thermal welding with the biowelder 121. In addition, at the same time, the tank 51 side of the tube 120 constituting the branch path 45, filled with the culture supernatant liquid 29, and the open end side of the flow-in tube 54 of the new empty tank 51 are sterilely connected to each other by the thermal welding with the biowelder 121.

In this way, the tube 120 constituting the branch path 45 and the flow-in tube 54 connected to the inlet port 53 of the tank 51 for the culture supernatant liquid 29 may be sterilely connected to each other by the thermal welding. According to this aspect, it is possible to further reduce the concern of contamination of the culture supernatant liquid 29. The sterile connection by the sterile connector 52 or the like and the sterile connection by the thermal welding may be performed in combination.

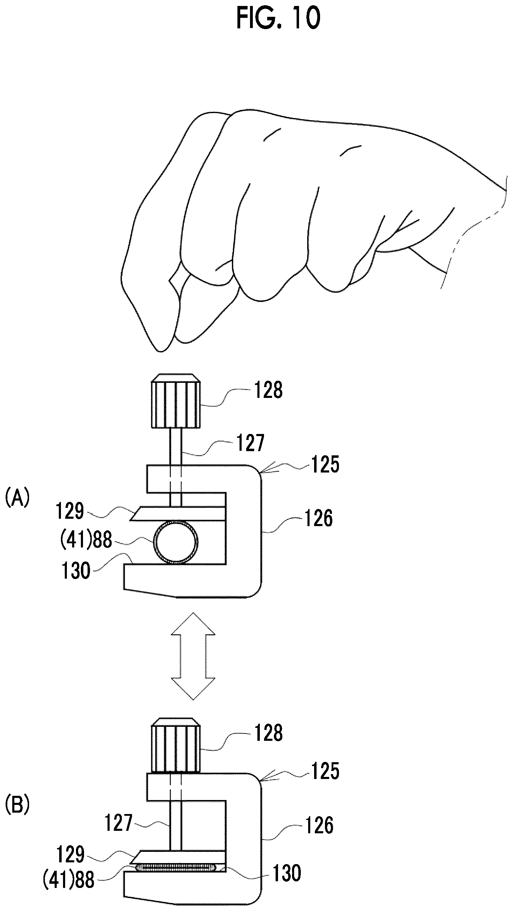

Although the pinch valves 49, 57, and 58 are mentioned as the no liquid-contact type valve, the present invention is not limited thereto. As an example, a screw-type tube clamp 125 shown in Fig. 10 may be used instead of the pinch valve 49 and the like.

Fig. 10 shows a case in which the screw-type tube clamp 125 is used instead of the pinch valve 49. In Fig. 10, the screw-type tube clamp 125 has a substantially U-shaped holder 126 and a screw 127. A head portion of the screw 127 is a pinch portion 128 for a user to pinch with a finger. A rectangular parallelepiped valve body 129 is attached to a distal end of the screw 127. The tube 88 constituting the coupling path 41 is set in the holder 126. The tube 88 is held in a state of being sandwiched between the valve body 129 and an action surface 130 of the holder 126.

In a state shown in (A), in which the screw 127 is loosened to the upper end, the tube 88 is not flattened by the valve body 129, and thus the coupling path 41 is open. Therefore, in the case of (A), the culture supernatant liquid 29 flows from the culture section 10 to the purification section 12 through the coupling path 41. On the other hand, in a state shown in (B), in which the screw 127 is tightened to the lower end, the tube 88 is flattened by the valve body 129 and the action surface 130, and thus the coupling path 41 is blocked. Therefore, in the case of (B), the flow of the culture supernatant liquid 29 from the culture section 10 to the purification section 12 through the coupling path 41 is blocked.

As described above, the no liquid-contact type valve may be a non-electrified type such as the screw-type tube clamp 125. As the tube clamp, in addition to the screw type, a crocodile mouth type, a roller type, a ratchet type, or the like can be adopted.

In a case where a non-electrified no liquid-contact type valve such as the screw-type tube clamp 125 is used, an indicator such as a warning light may be provided in the purification section 12, and the user may be notified of the occurrence of the malfunction in the purification section 12 by the indicator. In this case, the chromatography devices 70 to 72 may not issue the error signal to the purification section control unit 112.

### [Second embodiment]

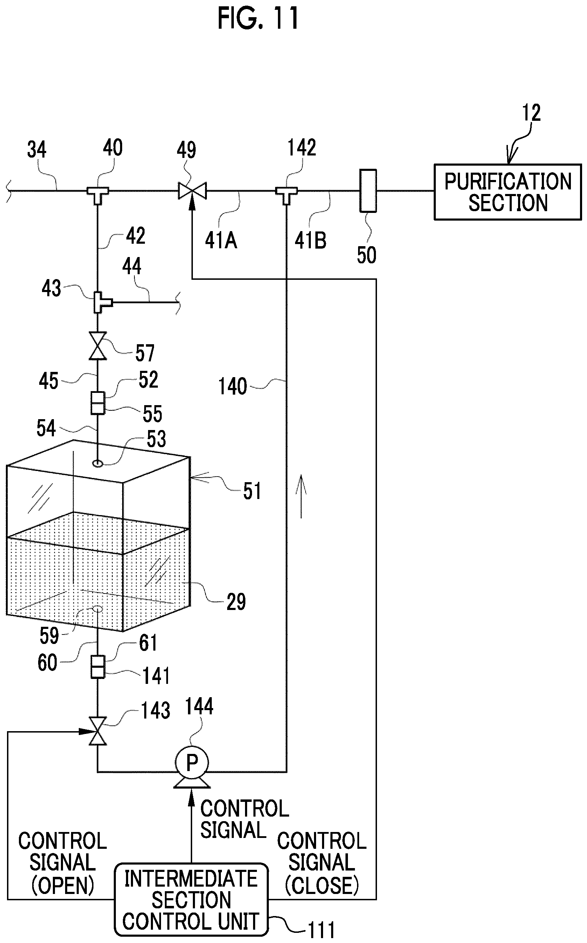

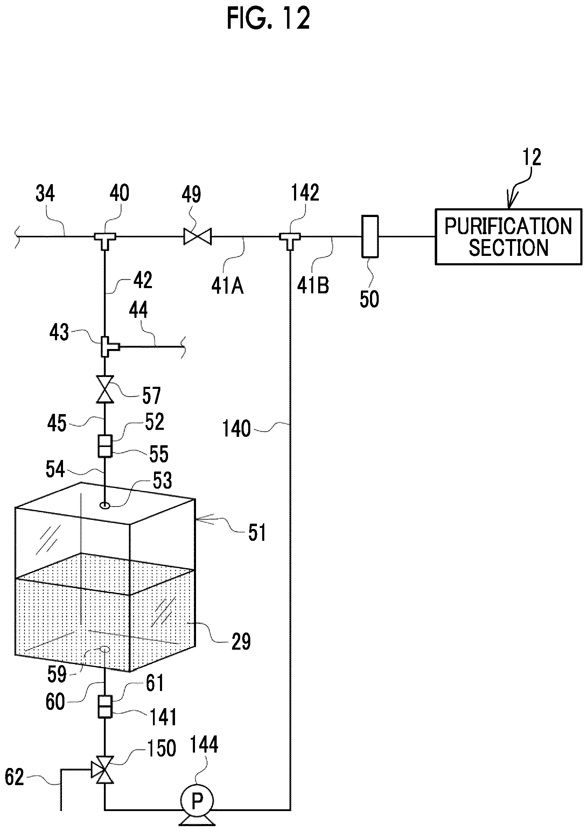

In the above-described first embodiment, the culture supernatant liquid 29 stored in the tank 51 is discarded, but the present disclosure is not limited thereto. As an example shown in Figs. 11 and 12, the culture supernatant liquid 29 stored in the tank 51 may flow into the purification section 12.

In Fig. 11, a return flow passage 140 is sterilely connected to a discharge tube 60 connected to a discharge port 59 of the tank 51 through a sterile connector 141, instead of the waste liquid path 62 of the first embodiment described above. The return flow passage 140 is connected to coupling paths 41A and 41B through a three-way joint 142. The three-way joint 142 is provided between the pinch valve 49 and the sterile filter 50. Therefore, the coupling path 41 is divided into an upstream-side coupling path 41A having the pinch valve 49 and a downstream-side coupling path 41B having the sterile filter 50.

A pinch valve 143 and a delivery pump 144 are provided in the return flow passage 140. The pinch valve 143 has the same configuration as the pinch valve 49 or the like. A control signal is transmitted from the intermediate section control unit 111 to the pinch valve 143. The pinch valve 143 opens and closes a tube constituting the return flow passage 140 in response to the control signal. The delivery pump 144 sends out the culture supernatant liquid 29 temporarily stored in the tank 51 toward the coupling path 41B and the purification section 12.

The intermediate section control unit 111 transmits the control signal (close) to the pinch valve 49, and then transmits the control signal (open) to the pinch valve 143. In addition, the intermediate section control unit 111 transmits the control signal to the delivery pump 144 to operate the delivery pump 144. As a result, the culture supernatant liquid 29 stored in the tank 51 flows into the purification section 12 through the discharge port 59, the discharge tube 60, the return flow passage 140, and the coupling path 41B.

Fig. 12 shows an example in which a liquid-contact type three-way valve 150 is provided in the return flow passage 140, instead of the pinch valve 143, and the waste liquid path 62 is connected to the liquid-contact type three-way valve 150. In this case, the intermediate section control unit 111 transmits the control signal (close) to the pinch valve 49, and then transmits the control signal for allowing the return flow passage 140 side to be open in the liquid-contact type three-way valve 150. In addition, the intermediate section control unit 111 transmits the control signal to the delivery pump 144 to operate the delivery pump 144. As a result, the culture supernatant liquid 29 stored in the tank 51 flows into the purification section 12. In a case where the culture supernatant liquid 29 stored in the tank 51 is discarded, the intermediate section control unit 111 transmits the control signal for allowing the liquid-contact type three-way valve 150 to open the waste liquid path 62 side.

As described above, in the second embodiment, the temporarily stored culture supernatant liquid 29 is allowed to flow into the purification section 12 from the discharge port 59 provided in the tank 51. Therefore, the culture supernatant liquid 29 temporarily stored in the tank 51 can be effectively used without waste.

In order to prevent the culture supernatant liquid 29 in which the quality of the antibody 18 has deteriorated from flowing into the purification section 12, it is preferable to measure a retention time of the culture supernatant liquid 29 in the tank 51, and to discard the culture supernatant liquid 29 in which the retention time exceeds a setting time, thereby not allowing to flow into the purification section 12. In addition, in a case where there is little advantage in allowing the culture supernatant liquid 29 to flow into the purification section 12 and reusing the culture supernatant liquid 29, such as a case in which the amount of the culture supernatant liquid 29 stored in the tank 51 is extremely small, the culture supernatant liquid 29 may be discarded without allowing the culture supernatant liquid 29 to flow into the purification section 12.

Whether or not the culture supernatant liquid 29 is allowed to flow into the purification section 12 may be determined according to the amount of the culture supernatant liquid 29 stored in the tank 51, and the purification ability of the purification section 12. Specifically, in a case where the amount of the culture supernatant liquid 29 stored in the tank 51 is an amount that can be treated by the purification ability of the purification section 12 within a predetermined time, the culture supernatant liquid 29 flows into the purification section 12. On the other hand, in a case where the amount of the culture supernatant liquid 29 stored in the tank 51 is an amount that cannot be treated by the purification ability of the purification section 12 within a predetermined time, the culture supernatant liquid 29 is discarded without being allowed to flow into the purification section 12.

### Examples

Hereinafter, Examples of the technology of the present disclosure will be described.

In the present example, the antibody-producing cells 17 derived from CHO cells, which produced the antibody 18, were cultured by perfusion culture in the culture tank 14 until the concentration thereof reached 12.0 × 10⁷ cells/mL (> 8.0 × 10⁷ cells/mL). Thereafter, immediately before a timing that the concentration reached 12.0 × 10⁷ cells/mL, the culture section 10 and the purification section 12 were connected to each other by a coupling line such as the coupling paths 34 and 41. After 5 days of operation in a state in which the culture section 10 and the purification section 12 were coupled to each other in this manner, the culture supernatant liquid 29 was allowed to flow into the tank 51 for 2 days, assuming that a malfunction occurred in the purification section 12. The evaluation was performed for each of Examples by changing various conditions such as the amount of the culture liquid 16 in the culture tank 14, the accommodation capacity of the tank 51, and the presence or absence of the sterile filter 50.

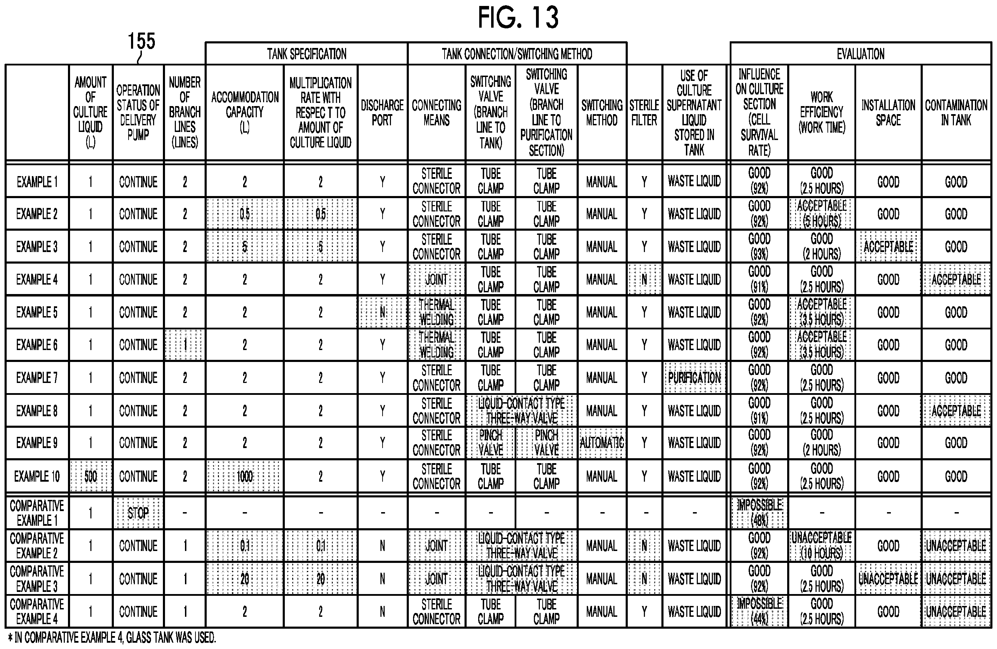

The results are shown in a table 155 of Fig. 13. The amount of the culture liquid 16 in the culture tank 14 was 1 L (Examples 1 to 9 and Comparative Examples 1 to 4) or 500 L (Example 10).

A specific experimental method in a case where the amount of the culture liquid 16 was 1 L was as follows.
1) 1 L of the culture liquid 16 was charged into the culture tank 14 having an accommodation capacity of 2 L, which was manufactured by ABLE Corporation & Biott Corporation.
2) The antibody-producing cells 17 derived from CHO cells were seeded in the culture liquid 16 with a concentration of 0.05 × 10⁷ cells/mL.
3) The stirring blade 25 was rotated at a rotation speed of 180 rotations per minute (rpm), and air and carbon dioxide were supplied from the gas supply path 20 at the upper part of the culture tank 14 at 19 mL/min and 1 mL/min, respectively; in addition, gas containing oxygen was supplied from the sparger 23 at the bottom of the culture tank 14 such that the oxygen concentration in the culture liquid 16 was 80%.
4) The antibody-producing cells 17 were cultured in the culture tank 14 while continuously supplying the culture liquid 16 from the culture liquid supply path 19 to the culture tank 14 at a perfusion ratio of 1.0 vessel volume per day (also referred to as vvd); thereafter, two days after the seeding, the culture liquid 16 was continuously filtered using ATF2 manufactured by Repligen Corporation as the filter 15, thereby obtaining the culture supernatant liquid 29.
5) The perfusion ratio was changed to 2.0 vvd on the 5th day of the seeding.
6) In a case where the concentration of the antibody-producing cells 17 in the culture liquid 16 reached 12.0 × 10⁷ cells/mL, a cell breeding treatment of supplying the culture liquid 16 while a part of the culture liquid 16 was taken out to maintain the concentration was performed; the cell bleed treatment was performed by feedback-controlling the amount of the culture liquid 16 to be taken out while monitoring the capacitance of the culture liquid 16 with a sensor manufactured by Aber Co., Ltd.
7) At the same time as the cell breeding treatment of 6) described above, the coupling paths 34 and 41 connected to the outlet 33 of ATF2 manufactured by Repligen Corporation were connected to the purification section 12, and the culture supernatant liquid 29 was continuously purified by the chromatography devices 70 to 72 of the purification section 12.
8) After maintaining the above-described state of 7) for 5 days, assuming that a malfunction occurred in the purification section 12, the culture supernatant liquid 29 was allowed to flow into the tank 51 for 2 days while the operation of the delivery pump 35 was continued; the concentration of the antibody 18 in the culture supernatant liquid 29 at this time was measured by Cedex Bio manufactured by Roche Bangladesh Limited, and was approximately 1.5 g/L to 2.0 g/L (> 1.0 g/L or more) over 5 days. In Comparative Example 1, since the tank 51 was not installed, the operation of the delivery pump 35 was stopped as a measure in a case where the malfunction occurred in the purification section 12.

A specific experimental method in a case where the amount of the culture liquid 16 was 500 L was the same as the conditions in a case where the amount of the culture liquid 16 was 1 L, except that the culture tank 14 was Hypeforma 500 manufactured by Thermo Fisher Scientific Inc., the 500 L of the culture liquid 16 was charged into the culture tank 14, the rotation speed of the stirring blade 25 was set to 150 rpm, air and carbon dioxide were supplied from the gas supply path 20 at the upper part of the culture tank 14 at 4.75 L/min and 0.25 L/min, respectively, and ATF10 manufactured by Repligen Corporation was used as the filter 15.

Evaluation items were the influence on the culture section 10, the work efficiency, the installation space, and the contamination in the tank 51.

The influence on the culture section 10 is the most important item. The influence on the culture section 10 was specifically evaluated based on a cell survival rate (viability) of the antibody-producing cells 17 in the culture liquid 16 in the culture tank 14 after allowing the culture supernatant liquid 29 to flow into the tank 51 for 2 days in 8) described above. In a case where the cell survival rate was 85% or more, the evaluation was "good", and in a case where the cell survival rate was less than 85%, the evaluation was "impossible".

The cell survival rate was measured by extracting the culture liquid 16 in the culture tank 14 after allowing the culture supernatant liquid 29 to flow into the tank 51 for 2 days, and then introducing the extracted culture liquid 16 into Cell Viability Analyzer Vi-cell XR manufactured by Beckman Coulter, Inc. Vi-cell XR2.04 was used as Vi-cell software, and parameters during the measurement were set as follows.
Min diameter: 6 µm
Max diameter: 50 µm
Dilution: in a case where the concentration of the antibody-producing cells 17 in the culture liquid 16 was less than 1.0 × 10⁷ cells/mL, the sample was not diluted and Dilution was set to 1;
in a case where the concentration of the antibody-producing cells 17 in the culture liquid 16 was 1.0 × 10⁷ cells/mL or more, the sample was diluted 10 times and Dilution was set to 10.
Cell brightness: 85%
Cell sharpness: 100
Viable cell spot brightness: 75%
Viable cell spot area: 5%
Minimum circularity: 0
Decluster degree: Medium

The work efficiency is the next most important item after the influence on the culture section 10. The work efficiency was evaluated based on work time required for switching work of the pinch valve 49 or the like after the malfunction occurred in the purification section 12; the connection work, the switching work, the replacement work of the tank 51; and the discarding work from the discharge port 59. The work time was evaluated as "good" for shorter than 3 hours, "acceptable" for 3 hours or longer and shorter than 6 hours, and "unacceptable" for 6 hours or longer, in 48 hours for 2 days.

The installation space was evaluated as "good" in a case where the installation area of the tank 51 was less than 1.5 times the installation area of the culture tank 14, "acceptable" in a case of being 1.5 times or more and less than 2.5 times, and "unacceptable" in a case of being 2.5 times or more.

The contamination in the tank 51 is not so important in a case where the culture supernatant liquid 29 temporarily stored in the tank 51 is discarded as waste liquid, but is important in a case where the culture supernatant liquid 29 temporarily stored in the tank 51 is allowed to flow into the purification section 12 and is re-used as in the above-described second embodiment. The contamination in the tank 51 was evaluated as "good" in a case where the contamination did not occur, "acceptable" in a case where the contamination occurred, and "unacceptable" in a case where the contamination was particularly severe.

Example 1 was an example in which the amount of the culture liquid 16 in the culture tank 14 was 1 L, the number of the branch lines connected to the tank 51, such as the branch path 45, was 2, the accommodation capacity of the tank 51 was 2 L, and the magnification of the accommodation capacity of the tank 51 to the amount of the culture liquid 16 was 2. In addition, Example 1 was an example in which the tank 51 was provided with the discharge port 59, and the connecting means between the branch line such as the branch path 45 and the tank 51 was the sterile connector 52. In addition, Example 1 was an example in which both the switching valve provided in the branch line such as the branch path 45 (referred to as "Switching valve (branch line to tank)" in the table 155) and the switching valve provided on the coupling line such as the coupling path 41 (referred to as "Switching valve (branch line to purification section)" in the table 155) were tube clamps such as the screw-type tube clamp 125 shown in Fig. 10, and the switching method of the switching valve was set to manual. Furthermore, Example 1 was an example in which the sterile filter 50 was provided, and the use of the culture supernatant liquid 29 stored in the tank 51 was for the waste liquid. In Example 1, all evaluations of the influence on the culture section 10, the work efficiency, the installation space, and the contamination in the tank 51 were "good".

Example 2 was the same as in Example 1, except that the accommodation capacity of the tank 51 was 0.5 L and the magnification of the accommodation capacity of the tank 51 to the amount of the culture liquid 16 was 0.5 times. In Example 2, only the evaluation on the work efficiency was "acceptable", and the rest were "good". The reason why the work efficiency was evaluated as "acceptable" is that the accommodation capacity of the tank 51 was smaller than that in Example 1, and thus the switching work, the replacement work, and the discarding work of the tank 51 were delayed. In a case where the accommodation capacity of the tank 51 was set to 0.2 L and the magnification of the accommodation capacity of the tank 51 to the amount of the culture liquid 16 was set to 0.2 times, the same evaluation results as in Example 2 were obtained.

Example 3 was the same as in Example 1, except that the accommodation capacity of the tank 51 was 5 L and the magnification of the accommodation capacity of the tank 51 to the amount of the culture liquid 16 was 5 times. In Example 3, only the evaluation on the installation space was "acceptable", and the rest were "good". The reason why the installation space was evaluated as "acceptable" is that the installation area of the tank 51 was simply increased because the accommodation capacity of the tank 51 was 2.5 times (10 times in Example 2) that of Example 1. In terms of the work efficiency, the working time of Example 1 was 2.5 hours, whereas the working time of Example 3 was improved to 2 hours because the accommodation capacity of the tank 51 was increased. In a case where the accommodation capacity of the tank 51 was set to 10 L and the magnification of the accommodation capacity of the tank 51 to the amount of the culture liquid 16 was set to 10 times, the same evaluation results as in Example 3 were obtained.

Example 4 was the same as in Example 1, except that the connecting means between the branch line and the tank 51 was a joint instead of the sterile connector 52 and the like, and the sterile filter 50 was not provided. In Example 4, only the evaluation on the contamination in the tank 51 was "acceptable", and the rest were "good". The reason why the contamination in the tank 51 was evaluated as "acceptable" is that the branch line and the tank 51 were not sterilely connected to each other and the sterile filter 50 did not prevent the invasion of bacteria or the like from the purification section 12.

Example 5 was the same as in Example 1, except that the discharge port 59 was not provided in the tank 51, and the connecting means between the branch line and the tank 51 was not the sterile connector 52 or the like but the thermal welding shown in Figs. 8 and 9. In Example 5, only the evaluation on the work efficiency was "acceptable", and the rest were "good". The reason why the work efficiency was evaluated as "acceptable" is that there was no way to discard the culture supernatant liquid 29 stored in the tank 51, and thus it was necessary to exchange the tank 51 with a new empty tank 51 by the method shown in Fig. 9 each time the tank 51 was filled with the culture supernatant liquid 29.

Example 6 was the same as in Example 1, except that the number of the branch lines connected to the tank 51 was 1 instead of 2, and the connecting means between the branch line and the tank 51 was not the sterile connector 52 or the like but the thermal welding shown in Figs. 8 and 9. In Example 6, only the evaluation on the work efficiency was "acceptable", and the rest were "good". The reason why the work efficiency was evaluated as "acceptable" is that the number of the branch lines connected to the tank 51 was 1, and only one tank 51 could be installed, and the culture supernatant liquid 29 had to be frequently discarded because the tank 51 could not be switched to another tank 51.

Example 7 was the same as in Example 1, except that the use of the culture supernatant liquid 29 stored in the tank 51 was not for the waste liquid but for the purification. In Example 7, same as Example 1, all evaluations of the influence on the culture section 10, the work efficiency, the installation space, and the contamination in the tank 51 were "good".

Example 8 was the same as in Example 1, except that, as the switching valve provided in the branch line and the switching valve provided in the coupling line, the liquid-contact type three-way valve was used in combination. In Example 8, only the evaluation on the contamination in the tank 51 was "acceptable", and the rest were "good". The reason why the contamination in the tank 51 was evaluated as "acceptable" is that the liquid-contact type valve was used instead of the tube clamp (no liquid-contact type valve).

Example 9 was the same as in Example 1, except that, as the switching valve provided in the branch line and the switching valve provided in the coupling line, the pinch valves 49, 57, 58, and the like shown in Fig. 2 were used, and the switching method of the switching valve was set to automatic. In Example 9, same as Example 1, all evaluations of the influence on the culture section 10, the work efficiency, the installation space, and the contamination in the tank 51 were "good". In terms of the work efficiency, the working time of Example 1 was 2.5 hours, whereas the working time of Example 9 was improved to 2 hours due to the automation.

Example 10 was an example in which the amount of the culture liquid 16 in the culture tank 14 was 500 L and the accommodation capacity of the tank 51 was 1,000 L. The other conditions were the same as in Example 1. In Example 10, same as Example 1, all evaluations of the influence on the culture section 10, the work efficiency, the installation space, and the contamination in the tank 51 were "good".

As described above, Comparative Example 1 was an example in which the tank 51 was not installed, and the operation of the delivery pump 35 was stopped as a measure in a case where the malfunction occurred in the purification section 12. In Comparative Example 1, the influence on the culture section 10 was evaluated as "unacceptable". The reason why the influence on the culture section 10 was evaluated as "unacceptable" is that, since the operation of the delivery pump 35 was stopped, the operation of the culture section 10 was stopped, which caused a large amount of cell death.

Comparative Example 2 was an example in which the accommodation capacity of the tank 51 was 0.1 L, the magnification of the accommodation capacity of the tank 51 to the amount of the culture liquid 16 was 0.1 times, the connection means between the branch line and the tank 51 was a j oint, the switching valve provided in the branch line and the switching valve provided in the coupling line were liquid-contact type three-way valves, and the sterile filter 50 was not provided. The other conditions were the same as in Example 1. In Comparative Example 2, the influence on the culture section 10 and the installation space were evaluated as "good" as in Example 1, but the work efficiency and the contamination in the tank 51 were evaluated as "unacceptable". The reason why the work efficiency was evaluated as "unacceptable" is that the accommodation capacity of the tank 51 was extremely small as 1/20 of that in Example 1, and thus the switching work, the replacement work, and the discarding work of the tank 51 were greatly delayed. The reason why the contamination in the tank 51 was evaluated as "unacceptable" is that the branch line and the tank 51 were not sterilely connected to each other, the sterile filter 50 did not prevent the invasion of bacteria or the like from the purification section 12, and the liquid-contact type valve was used instead of the tube clamp (no liquid-contact type valve).

Comparative Example 3 was the same as in Comparative Example 2, except that the accommodation capacity of the tank 51 was 20 L and the magnification of the accommodation capacity of the tank 51 to the amount of the culture liquid 16 was 20 times. In Comparative Example 3, the influence on the culture section 10 and the work efficiency were evaluated as "good" as in Example 1, but the installation space and the contamination in the tank 51 were evaluated as "unacceptable". The reason why the installation space was evaluated as "unacceptable" is that, same as in Example 3, the installation area of the tank 51 was simply increased because the accommodation capacity of the tank 51 was 10 times that of Example 1. The reason why the contamination in the tank 51 was evaluated as "not possible" is the same as in Comparative Example 2.

Comparative Example 4 was the same as in Example 1, except that, as the tank 51, the glass tank as disclosed in JP2017-515501A was used instead of the single-use tank. In Comparative Example 4, the work efficiency and the installation space were evaluated as "good" as in Example 1, but the influence on the culture section 10 and the contamination in the tank 51 were evaluated as "unacceptable". The reason why the influence on the culture section 10 and the contamination in the tank 51 were evaluated as "unacceptable" is that, since the tank 51 was made of glass, the culture supernatant liquid 29 in the tank 51 was contaminated in the first place, and this contamination in the tank 51 affects the culture section 10.

From Examples 1 to 10 and Comparative Examples 1 to 4, it was found that the accommodation capacity of the tank 51 is suitably set to 0.2 times to 10 times the amount of the culture liquid 16 in the culture tank 14. In addition, it was found that the work efficiency was better in a case where the number of the branch lines was a plurality of lines rather than one line, and the work efficiency was better in a case where the discharge port 59 was provided in the tank 51. In addition, it was found that, in a case where the connection means between the branch line and the tank 51 was the sterile connector 52 or the like or the thermal welding, and the sterile filter 50 was provided, the contamination in the tank 51 could be effectively prevented. Furthermore, it was found that, in a case where the flow passage of the culture supernatant liquid 29 was switched from the coupling line to the branch line using the switching valve, the contamination in the tank 51 could be effectively prevented by using the no liquid-contact type valve such as the tube clamp or the pinch valve. Furthermore, it was also found that the contamination in the culture section 10 and the tank 51 could be effectively prevented by using the single-use tank 51.

The product is not limited to the antibody 18. The product is not particularly limited as long as it is the active pharmaceutical ingredient 79 of a biopharmaceutical, and may be a protein, a peptide, a nucleic acid (DNA or ribonucleic acid (RNA)), a lipid, a virus, a virus subunit, a virus-like particle, or the like.

In each of the above-described embodiments, for example, as hardware structures of processing units that execute various kinds of processing, such as the culture section control unit 110, the intermediate section control unit 111, and the purification section control unit 112, various processors shown below can be used. The various processors include, for example, the CPU 97 which is a general-purpose processor executing software (control program 105) to function as various processors as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of the various types of processors or may be configured by a combination of two or more processors of the same type or different types (for example, a combination of a plurality of FPGAs and/or a combination of a CPU and an FPGA). In addition, a plurality of processing units may be configured by one processor.

As an example of configuring the plurality of processing units with one processor, first, there is a form in which one processor is configured by a combination of one or more CPUs and software and the processor functions as the plurality of processing units, as represented by computers such as a client and a server. A second example of the configuration is an aspect in which a processor that implements the functions of the entire system including a plurality of processing units using one integrated circuit (IC) chip is used. A representative example of the aspect is a system-on-chip (SoC). In this manner, various processing units are configured by using one or more of the above-described various processors as hardware structures.

In addition, more specifically, an electric circuit (circuitry) in which circuit elements, such as semiconductor elements, are combined can be used as the hardware structure of these various processors.

In the technology of the present disclosure, the above-described various embodiments and/or various modification examples can be appropriately combined. It is needless to say that the technique of the present disclosure is not limited to each of the embodiments described above and various configurations can be employed without departing from the gist. Furthermore, the technology of the present disclosure extends to a storage medium that non-transitorily stores a program in addition to the program.

The contents described and shown above are detailed descriptions of portions according to the technology of the present disclosure and are merely examples of the technology of the present disclosure. For example, the above description of the configurations, functions, operations, and effects is the description of examples of the configurations, functions, operations, and effects of portions related to the technology of the present disclosure. Therefore, unnecessary portions may be deleted or new elements may be added or replaced in the above descriptions and illustrations without departing from the gist of the technology of the present disclosure. In addition, in order to avoid complication and facilitate understanding of portions according to the technology of the present disclosure, description related to common technical knowledge or the like that does not need to be particularly described for enabling implementation of the technology of the present disclosure is omitted in the contents described and shown above.

In the present specification, "A and/or B" has the same meaning as "at least one of A or B". That is, the "A and/or B" means only A, only B, or a combination of A and B. In addition, in the present specification, in a case where three or more matters are expressed by being connected by "and/or", the same concept as "A and/or B" is applied.

All documents, patent applications, and technical standards described in the present specification are incorporated herein by reference to the same extent as in a case of being specifically and individually noted that individual documents, patent applications, and technical standards are incorporated by reference.

## Claims

1. A method for producing an active pharmaceutical ingredient of a biopharmaceutical, the method comprising:
connecting, through a coupling line, a culture section where a culture step, in which cells are cultured in a culture liquid stored in a culture tank and the cells are removed from the culture liquid to obtain a culture supernatant liquid containing a product of the cells, is performed and a purification section where a purification step, in which the product is purified from the culture supernatant liquid to obtain an active pharmaceutical ingredient of a biopharmaceutical, is performed, to allow the culture supernatant liquid to flow into the purification section from the culture section through the coupling line;
connecting a flexible single-use tank having an accommodation capacity of 0.2 times to 10 times an amount of the culture liquid in the culture tank to a branch line provided between the culture section and the purification section; and
temporarily storing the culture supernatant liquid in the tank.

2. The method for producing an active pharmaceutical ingredient of a biopharmaceutical according to claim 1,
wherein all flow passages of the culture supernatant liquid from the culture section to the tank are sterilely connected.

3. The method for producing an active pharmaceutical ingredient of a biopharmaceutical according to claim 2,
wherein both a connecting portion of the branch line with the tank and a connecting portion of the tank with the branch line are sterile connectors, and
the branch line and the tank are sterilely connected to each other through the sterile connectors.

4. The method for producing an active pharmaceutical ingredient of a biopharmaceutical according to claim 2 or 3,
wherein a tube constituting the branch line and a flow-in tube connected to an inlet port of the tank for the culture supernatant liquid are sterilely connected to each other by thermal welding.

5. The method for producing an active pharmaceutical ingredient of a biopharmaceutical according to any one of claims 1 to 4,
wherein the culture supernatant liquid flowing into the purification section is filtered by a sterile filter provided downstream of the coupling line with respect to a connecting portion of the branch line.

6. The method for producing an active pharmaceutical ingredient of a biopharmaceutical according to any one of claims 1 to 5,
wherein the amount of the culture liquid in the culture tank is 50 L or more and 5,000 L or less.

7. The method for producing an active pharmaceutical ingredient of a biopharmaceutical according to any one of claims 1 to 6,
wherein the temporarily stored culture supernatant liquid is discarded from a discharge port provided in the tank.

8. The method for producing an active pharmaceutical ingredient of a biopharmaceutical according to any one of claims 1 to 7,
wherein the temporarily stored culture supernatant liquid is allowed to flow into the purification section from a discharge port provided in the tank.

9. The method for producing an active pharmaceutical ingredient of a biopharmaceutical according to any one of claims 1 to 8,
wherein a plurality of the branch lines are provided between the culture section and the purification section.

10. The method for producing an active pharmaceutical ingredient of a biopharmaceutical according to claim 9,
wherein, in a case where the tank connected to one of the plurality of the branch lines is filled with the culture supernatant liquid, the tank connected to another branch line is switched for use.

11. The method for producing an active pharmaceutical ingredient of a biopharmaceutical according to any one of claims 1 to 10,
wherein, in a case where a malfunction occurs in the purification section, a flow passage of the culture supernatant liquid is switched from the coupling line to the branch line.

12. The method for producing an active pharmaceutical ingredient of a biopharmaceutical according to claim 11,
wherein the flow passage of the culture supernatant liquid is switched using a no liquid-contact type valve capable of switching the flow passage without coming into contact with the culture supernatant liquid.

13. The method for producing an active pharmaceutical ingredient of a biopharmaceutical according to claim 12,
wherein the flow passage of the culture supernatant liquid is switched using the no liquid-contact type valve provided in the branch line and the no liquid-contact type valve provided downstream of the coupling line with respect to a connecting portion of the branch line.

14. The method for producing an active pharmaceutical ingredient of a biopharmaceutical according to claim 12 or 13,
wherein the no liquid-contact type valve is a type in which a tube is sandwiched from an outside.

15. The method for producing an active pharmaceutical ingredient of a biopharmaceutical according to any one of claims 11 to 14,
wherein, in a case where a signal indicating that a malfunction has occurred is output from the purification section, control of switching the flow passage of the culture supernatant liquid from the coupling line to the branch line is performed.

16. The method for producing an active pharmaceutical ingredient of a biopharmaceutical according to any one of claims 1 to 15, further comprising:
measuring a flow rate of the culture supernatant liquid with a flowmeter provided upstream of the coupling line with respect to a connecting portion of the branch line; and
controlling the flow rate of the culture supernatant liquid based on a measurement result.

17. The method for producing an active pharmaceutical ingredient of a biopharmaceutical according to any one of claims 1 to 16,
wherein the purification section continuously purifies the culture supernatant liquid.

18. The method for producing an active pharmaceutical ingredient of a biopharmaceutical according to any one of claims 1 to 17,
wherein the product is an antibody.

19. The method for producing an active pharmaceutical ingredient of a biopharmaceutical according to claim 18,
wherein a concentration of the antibody in the culture supernatant liquid is 1.0 g/L or more.

20. The method for producing an active pharmaceutical ingredient of a biopharmaceutical according to any one of claims 1 to 19,
wherein a concentration of the cells in the culture liquid is 8.0 × 10⁷ cells/mL or more.

21. A system for producing an active pharmaceutical ingredient of a biopharmaceutical, the system comprising:
a culture section where a culture step, in which cells are cultured in a culture liquid stored in a culture tank and the cells are removed from the culture liquid to obtain a culture supernatant liquid containing a product of the cells, is performed;
a purification section where a purification step, in which the product is purified from the culture supernatant liquid to obtain an active pharmaceutical ingredient of a biopharmaceutical, is performed;
a coupling line which connects the culture section and the purification section, through which the culture supernatant liquid flows from the culture section to the purification section;
a branch line which is provided between the culture section and the purification section; and
a flexible single-use tank which is connected to the branch line, has an accommodation capacity of 0.2 times to 10 times an amount of the culture liquid in the culture tank, and temporarily stores the culture supernatant liquid.

22. An active pharmaceutical ingredient for a biopharmaceutical, which is produced by the method for producing an active pharmaceutical ingredient of a biopharmaceutical according to any one of claims 1 to 20.
